# EUROPEAN PATENT SPECIFICATION

(11) **EP 4 501 222 B1**
(45) Date of publication and mention of the grant of the patent: **23.07.2025**
(21) Application number: 23188773.8
(22) Date of filing: 31.07.2023
(51) Int. Cl.: A61B 5/024, A61B 5/0507, A61B 5/00, G01S 13/88, A61B 5/08

(54) **APPARATUS, RADAR SYSTEM, AND METHOD FOR ESTIMATING A HEART RATE**
VORRICHTUNG, RADARSYSTEM UND VERFAHREN ZUR SCHÄTZUNG EINER HERZFREQUENZ
APPAREIL, SYSTÈME RADAR ET PROCÉDÉ D'ESTIMATION DE FRÉQUENCE CARDIAQUE

(43) Date of publication of application: 05.02.2025
(73) Proprietor: Infineon Technologies AG, 85579 Neubiberg (DE)
(72) Inventor: LEUNG, Tsz Shing, Kowoloon (HK)
(74) Representative: 2SPL Patentanwälte PartG mbB

(56) References cited:
- CN-A- 112 716 462

## Description

### Field

The present disclosure relates to radar data processing. In particular, examples relate to an apparatus, a radar system, a method, and a program for estimating a heart rate.

### Background

Vital signs, such as a breathing rate and heart rate, are physical measurements of the body that can be used to monitor a general health status of a living being. While breathing rates and heart rates may be measured by various methods, such methods under certain circumstances might not be feasible. In particular, for a mostly static target, a high velocity resolution for detection of a breathing rate may be more easily obtained compared to a heart rate, which is more challenging. Also for radar systems, the detection of the heart rate faces many challenges, due to environmental noise, harmonic components of the breathing rate, and random body movement. Thus, there is a demand for improved radar data processing for heart rate detection.

CN 112716462 A discloses a narrow-beam millimeter wave human body heartbeat/respiration sign monitoring device with controllable irradiation direction. The direction of a main beam of a radar device can be changed by using a beam control mechanism for automatically searching a maximum position. This can be used for monitoring the heartbeat amplitude of the body surface of the human body according to the amplitude characteristic of the demodulated intermediate frequency signal phase of the radar receiver.

### Summary

This demand is satisfied by the subject matter of the independent claims. Further beneficial embodiments are given by the dependent claims.

According to a first aspect, the present disclosure relates to an apparatus for measuring a heart rate of a target in a field of view of a radar sensor. The apparatus comprises processing circuitry configured to determine a frequency spectrum corresponding to time-domain data based on a receive signal generated by the radar sensor. The processing circuitry is further configured to determine a breathing rate frequency based on a maximum amplitude of the frequency spectrum. Furthermore, the processing circuitry is configured to determine a plurality of section-frequency-spectra, each corresponding to a respective section of the time-domain data, based on the breathing rate frequency, and to determine an average of the section-frequency-spectra to obtain an averaged-frequency-spectrum. The processing circuitry is further configured to subtract the averaged-frequency-spectrum from the frequency spectrum to obtain a difference spectrum, and to estimate a heart rate of the target based on a maximum amplitude of the difference spectrum. According to a second aspect, the present disclosure relates to a radar system comprising an apparatus as described herein and a radar sensor configured to generate the receive signal.

According to a third aspect, the present disclosure relates to a method for measuring a heart rate of a target in a field of view of a radar sensor. The method comprises determining a frequency spectrum corresponding to time-domain data based on a receive signal generated by the radar sensor. The method further comprises determining a breathing rate frequency based on a maximum amplitude of the frequency spectrum. Furthermore, the method comprises determining a plurality of section-frequency-spectra, each corresponding to a respective section of the time-domain data, based on the breathing rate frequency, and further comprises determining an average of the section-frequency-spectra to obtain an averaged-frequency-spectrum. Furthermore, the method comprises subtracting the averaged-frequency-spectrum from the frequency spectrum to obtain a difference spectrum and estimating a heart rate of the target based on a maximum amplitude of the difference spectrum.

According to a fourth aspect, the present disclosure relates to a program having a program code for performing a method as described herein when the program is executed on a processor or programmable hardware.

### Brief description of the Figures

Some examples of apparatuses and/or methods will be described in the following by way of example only, and with reference to the accompanying figures, in which
- Fig. 1: schematically illustrates an exemplary apparatus for measuring a heart rate of a target in a field of view of a radar sensor;
- Fig. 2A: depicts an exemplary frequency spectrum;
- Fig. 2B: depicts an exemplary averaged-frequency-spectrum;
- Fig. 3: provides a flow chart of exemplary pre-processing steps that may be applied before determining the frequency spectrum;
- Fig. 4: provides a visual representation for exemplary processing steps for measuring a heart rate of a target in a field of view of a radar sensor;
- Fig. 5: depicts a range-time profile corresponding to time-domain data within a specified range bin of a range Fourier Transformation.
- Fig. 6: provides a flow chart of exemplary processing steps that may be applied for measuring a heart rate of a target in a field of view of a radar sensor;
- Fig. 7A: depicts an exemplary frequency spectrum and an exemplary averaged-frequency-spectrum plotted together;
- Fig. 7B: depicts an exemplary windowed frequency spectrum for a respective correlation operation;
- Fig. 7C: depicts an exemplary correlated frequency spectrum and an exemplary correlated averaged-frequency-spectrum generated by respective correlation operations;
- Fig. 8: provides a zoomed-in depiction of the heart rate component of an exemplary difference spectrum taken from an exemplary correlated frequency spectrum;
- Fig. 9: schematically illustrates an exemplary radar system for measuring a heart rate of a target in a field of view of a radar sensor; and
- Fig. 10: provides a flow chart of an exemplary method for measuring a heart rate of a target in a field of view of a radar sensor.

### Detailed Description

Some examples are now described in more detail with reference to the enclosed figures. However, other possible examples are not limited to the features of these embodiments described in detail. Other examples may include modifications of the features as well as equivalents and alternatives to the features. Furthermore, the terminology used herein to describe certain examples should not be restrictive of further possible examples.

Throughout the description of the figures same or similar reference numerals refer to same or similar elements and/or features, which may be identical or implemented in a modified form while providing the same or a similar function. The thickness of lines, layers and/or areas in the figures may also be exaggerated for clarification.

When two elements A and B are combined using an "or", this is to be understood as disclosing all possible combinations, i.e. only A, only B as well as A and B, unless expressly defined otherwise in the individual case. As an alternative wording for the same combinations, "at least one of A and B" or "A and/or B" may be used. This applies equivalently to combinations of more than two elements.

If a singular form, such as "a", "an" and "the" is used and the use of only a single element is not defined as mandatory either explicitly or implicitly, further examples may also use several elements to implement the same function. If a function is described below as implemented using multiple elements, further examples may implement the same function using a single element or a single processing entity. It is further understood that the terms "include", "including", "comprise" and/or "comprising", when used, describe the presence of the specified features, integers, steps, operations, processes, elements, components and/or a group thereof, but do not exclude the presence or addition of one or more other features, integers, steps, operations, processes, elements, components and/or a group thereof.

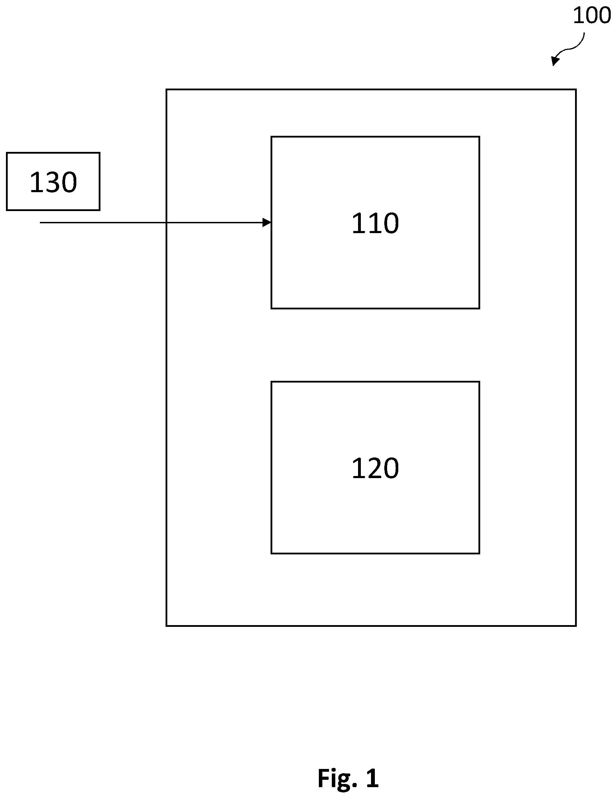

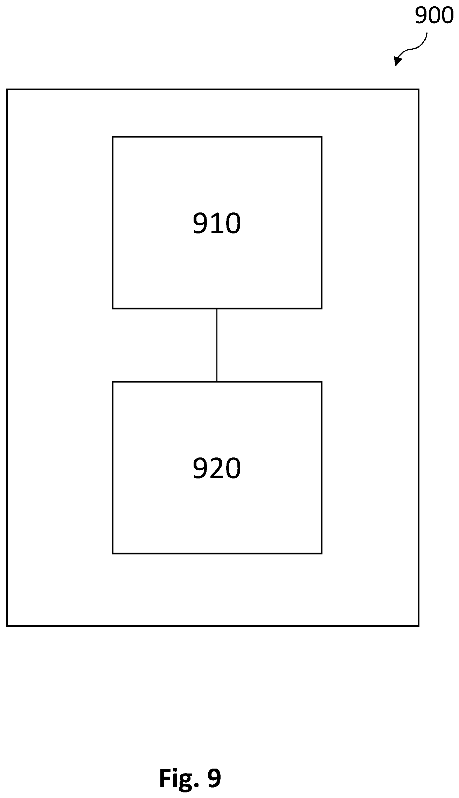

**Fig. 1** illustrates a block diagram of an example of an apparatus 100. The apparatus 100 is to be considered in the context of a radar sensor. For instance, the apparatus 100 may be integrated into a radar system comprising the radar sensor such as explained below with reference to Fig. 9 or may be external to the radar system. In the former case, the apparatus 100 may be external to or (e.g., partially or fully) integrated into the radar sensor. For instance, the apparatus 100 may be distributed between the radar sensor and a location external to the radar sensor.

The apparatus 100 comprises processing circuitry 110 and, optionally, interface circuitry 120. In case interface circuitry 120 is present, the interface circuitry 120 may be communicatively coupled (e.g., via a wired or wireless connection) to the processing circuitry 110, e.g., for data exchange between the interface circuitry 120 and the processing circuitry 110. The interface circuitry 120 may be any device or means for communicating or exchanging data. In case the apparatus 100 comprises the interface circuitry 120, the interface circuitry 120 may be configured to receive data indicating a receive signal of the radar sensor. For instance, the interface circuitry 120 may be communicatively coupled to the radar sensor or to a storage device storing the data. The interface circuitry 120 may receive the data, e.g., via a wired or wireless coupling to the radar sensor or the storage device.

Depending on the specific implementation, the apparatus 100 may dispense with the interface circuitry 120. For example, the processing circuitry 110 may determine said data. For instance, the processing circuitry 110 may be integrated into the radar sensor. The radar sensor may be any device that uses radio waves to, e.g., detect and locate objects. The radar sensor may be an, e.g., FMCW (frequency modulated continuous wave) radar sensor. For instance, the radar sensor may be configured to emit, by a transmitter, a radio frequency signal (Tx signal) into a field of view (a scene) of the radar sensor and receive, by a receiver, a reflection (echo; Rx signal) of the radar frequency signal. The radar sensor or an external device coupled to the radar sensor may generate radar data based on the received reflection of the radio frequency signal by, e.g., sampling the received reflection by means of an analog-to-digital converter (ADC).

The radio frequency signal (i.e. receive signal) may be in the form of a plurality of chirps, including parts of a received reflection signal which are correlated to respective emitted chirps. A chirp may be a radio frequency signal that varies in frequency over time. The frequency of the chirp may be swept over a specific frequency range, e.g., over the chirp bandwidth. For instance, the chirp may be a linearly modulated signal, i.e., a signal of which the frequency increases or decreases linearly over time.

The receive signal may be any type of signal that is generated in a receiver of the radar sensor. The receive signal may be an intermediate frequency, IF, signal, which may e.g., be created by mixing the reflection with a local oscillator signal at a specific frequency. The processing circuitry 110 may determine data indicating the receive signal by, e.g., sampling the receive signal and perform further processing of the data within the radar sensor. The processing circuitry 110 may optionally modify the sampled receive signal in a pre-processing step, e.g., for noise-reduction, DC-removal (direct current) or alike. For instance, the apparatus 100 may comprise memory configured to store such generated data.

The radar sensor may mix the received echo with a replica of the emitted signal using a mixer to produce an IF signal xIF(t) (e.g., a beat signal). The radar sensor may comprise one or more antennas to receive the reflected signal and the radar sensor may comprise an amplifier to receive the reflected signals from its antennas. The beat signal xIF(t) may be filtered with a low-pass filter (LPF) and then sampled by the ADC. The ADC may advantageously be capable of sampling the filtered beat signals xout(t) with a sampling frequency that is smaller than the frequency of the received signal received by the receiving antennas.

Alternatively, the processing circuitry 110 may partially determine the data. For instance, the processing circuitry 110 may determine a first part of the data, whereas at least one external processing circuitry may determine at least a second part of the data. The processing circuitry 110 and the external processing circuitry may, e.g., be connected within a distributed computing environment for jointly determining the data. In this case, the processing circuitry 110 may either be integrated into the radar sensor or may be external to the radar sensor. The processing circuitry 110 may receive the second part of the data, e.g., via an interface to the external processing circuitry such as interface circuitry 120, and further process the first and the second part of the data.

In another alternative, the processing circuitry 110 is partially integrated into the radar sensor and is partially external to the radar sensor. In such cases, the interface circuitry 120 is optional. The processing circuitry 110 may, for instance, comprise a first part (first processing circuitry) which is integrated into the radar sensor and a second part (second processing circuitry) which is external to the radar sensor. In this case, the determination of the data and/or further processing may be performed by the first and second part of the processing circuitry 110 in a distributed manner.

The processing circuitry 110 may be, e.g., a single dedicated processor, a single shared processor, or a plurality of individual processors, some of which or all of which may be shared, a digital signal processor (DSP) hardware, an application specific integrated circuit (ASIC), a microcontroller or a field programmable gate array (FPGA). The processing circuitry 110 may optionally be coupled to, e.g., read only memory (ROM) for storing software, random access memory (RAM) and/or non-volatile memory.

As part of the above-described radar system and its multiple components, the various embodiments of the apparatus 100 described herein are provided for determining a heart rate and a breathing rate of a target in the field of view of the radar sensor. For this, the apparatus 100 is configured to receive data based on the receive signal, including time-domain data 130, and perform processing steps based the time-domain data 130. The time-domain data 130 may be checked and/or processed into other forms of data before being used for generating a plurality of frequency spectra that may be analyzed for determining the heart rate and breathing rate of the target.

The processing circuitry 110 of the apparatus 100 is configured to determine a frequency spectrum corresponding to the time-domain data 130 and to determine a breathing rate frequency based on a maximum amplitude therein. The breathing rate frequency may be determined by an analysis of the frequency spectrum, including a search for and identification of a maximum amplitude of the frequency spectrum. The breathing rate frequency may be determined by identifying a frequency bin, within which the amplitude is at a maximum value for the entire frequency spectrum. The breathing rate frequency may be set to a value within a frequency range corresponding to the frequency bin, which may be equivalent to a center frequency within the frequency bin.

The frequency spectrum may take the form of a variety of frequency spectrum types. For example, the frequency spectrum may be an amplitude spectrum, wherein the frequency spectrum comprises a plurality of amplitudes, each amplitude corresponding to a respective frequency bin (or frequency component). In a particular example, the frequency spectrum may be a magnitude spectrum representing a magnitude or absolute value of complex amplitudes in a frequency domain. As such, the magnitude spectrum may provide information about an intensity or strength corresponding to a frequency component without considering phase information. Alternatively, the frequency spectrum may be a power spectrum representing a power of each frequency component. The frequency spectrum may also be a normalized spectrum, representing the amplitudes or powers of the frequency components scaled to a specific reference or normalized to a specific value. The various types of frequency spectra presented for the above-described frequency spectrum may also analogously apply to all other frequency spectra used in further processing.

The processing circuitry 110 is further configured to determine a plurality of section-frequency-spectra, each corresponding to a respective section of the time-domain data 130. Furthermore, the plurality of section-frequency spectra are determined based on the breathing rate frequency. For example, the time-domain data 130 may be divided into a plurality of sections, wherein each section corresponds to a time interval, which may be the same amount of time for each section. The time interval may be defined by the inverse of the breathing rate frequency. For example, the inverse of the breathing rate frequency may provide a time interval equivalent to an amount of time between successive breaths taken at a frequency equivalent to the breathing rate frequency. As such, each section of the time-domain data 130 may include a single breath at a similar time instance within the time interval.

The processing circuitry 110 is further configured to determine an average of the section-frequency-spectra to obtain an averaged-frequency-spectrum. Each of the section-frequency-spectra may comprise a common frequency range and a common number of frequency bins (e.g. a same resolution). The processing circuitry 110 may be configured to sum the respective amplitudes of each section-frequency-spectrum and divide the sum by the number of section-frequency-spectra. More specifically, the respective amplitude values of each section-frequency-spectrum that each correspond to a particular frequency bin, such as a frequency bin of an equivalent position or index, may be summed and divided by the number of section-frequency-spectra. This may also be done for each frequency bin of the frequency spectra, so that each frequency bin has associated therewith an averaged amplitude value. The plurality of averaged amplitude values, each associated with its respective frequency bin, may be used for determining the averaged-frequency-spectrum.

The processing circuitry 110 is configured to subtract the averaged-frequency-spectrum from the (original) frequency spectrum to obtain a difference spectrum. For the subtraction, the respective amplitude values of each frequency bin of the averaged-frequency-spectrum may be subtracted from the respective amplitude values of each frequency bin of the (original) frequency spectrum. For example, an amplitude value of a particular frequency bin of the averaged-frequency-spectrum may be subtracted from an amplitude value of an analogous frequency bin with an equivalent position or index of the (original) frequency spectrum. This may be done for each frequency bin of the averaged-frequency-spectrum and the (original) frequency spectrum to obtain a difference value for each frequency bin. The respective difference values may then be used to obtain a full difference spectrum. The difference spectrum may also be considered as a difference dataset with respective difference-in-amplitude values, wherein the difference dataset may be plotted according to the respective frequency bins to provide a difference (frequency) spectrum.

Furthermore, the processing circuitry 110 is configured to estimate a heart rate of the target based on a maximum amplitude of the difference spectrum. The heart rate may correspond to a specific heart rate frequency, which may correspond to the maximum amplitude of the difference spectrum. Similar to finding the breathing rate frequency based on a maximum amplitude, the heart rate frequency may be determined by an analysis of the difference spectrum, including a search for and identification of a maximum amplitude of the difference spectrum. The heart rate frequency may be determined by identifying a frequency bin, within which the amplitude is at a maximum value for the entire difference spectrum. The heart rate frequency may be determined as a value within a frequency range corresponding to the frequency bin, which may be equivalent to a center frequency within the frequency bin.

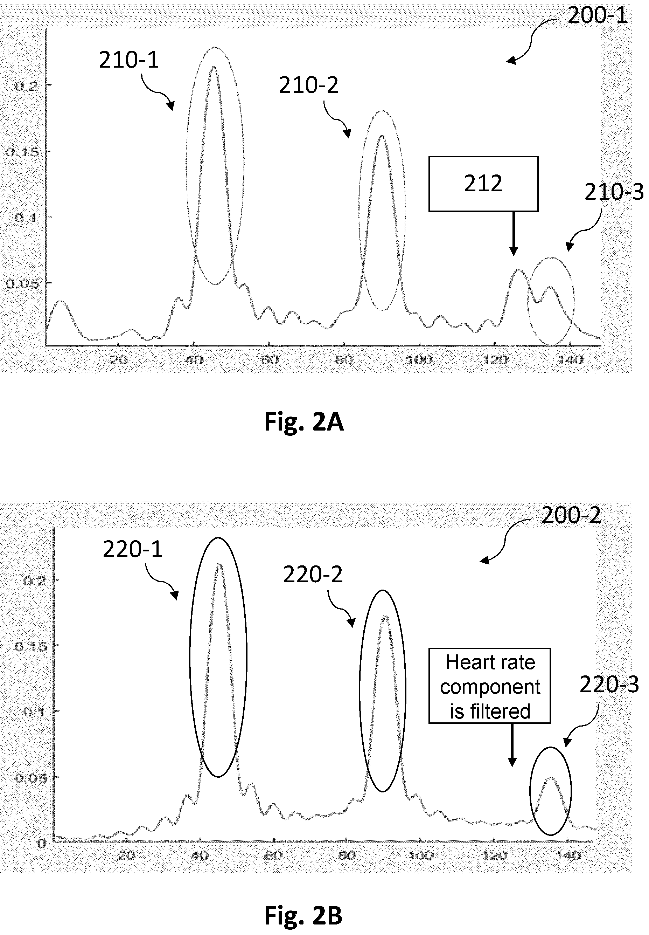

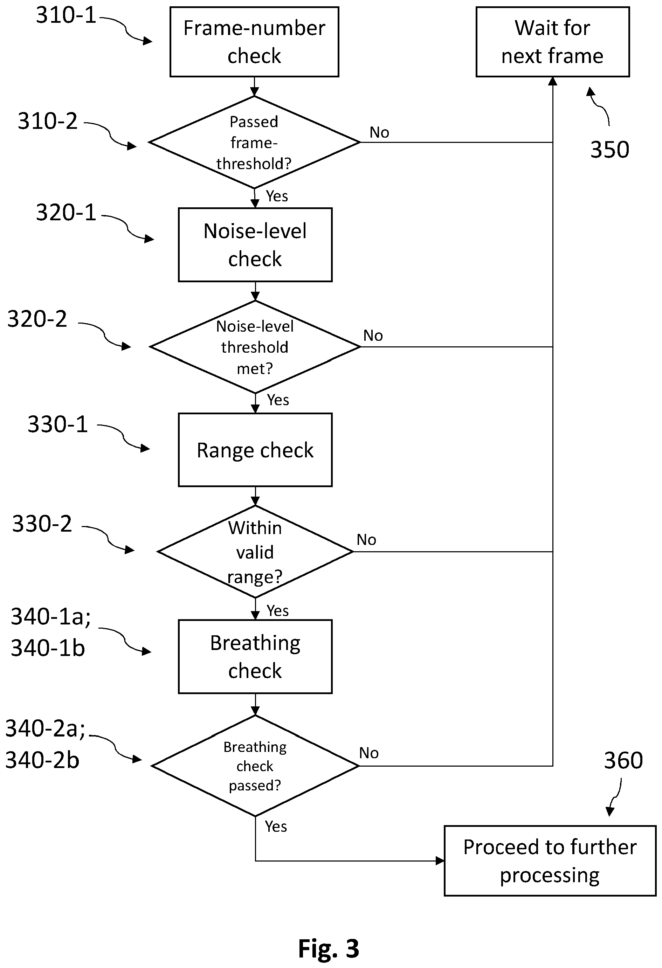

As such, the processing circuitry 110 is configured to obtain a heart rate frequency (e.g., estimate a heart rate) and obtain (i.e. determine) a breathing rate frequency. The heart rate frequency and breathing rate frequencies may each be used to provide health information related to the target in the field of view of the radar sensor. Such heart rate and breathing rate frequencies may also be recorded multiple times, which may performed over a period of days, weeks, months, or years to obtain long-term health information related to the target. The apparatus 100 may comprise other features that may improve the accuracy of the obtained breathing rate and/or heart rate frequencies. Such features will be discussed in greater depth with reference to Figs. 3 to 8. The features of the apparatus 100 previously discussed are also discussed in greater depth with illustrative examples with reference to Figs. 2A and 2B.

**Fig. 2A** depicts an exemplary frequency spectrum 200-1. The y-axis may correspond to an amplitude of the frequency. As previously described, the amplitude may correspond to either a magnitude or a power of the frequency spectrum, which may have been normalized or re-scaled with respect to a specific value. The x-axis of the frequency spectrum 200-1 may correspond to values for a frequency within a pre-specified frequency range. The frequencies of the x-axis may be measured in inverse minutes, which may be any arbitrary unit per minute (e.g. breaths per minute or beats per minute). The depicted inverse minutes are directly proportional to a scale measured in Hertz (Hz), e.g., s⁻¹ or inverse seconds.

The frequency spectrum 200-1 is shown with a first amplitude peak 210-1 corresponding to the maximum amplitude. The maximum amplitude peak 210-1 is located at a first frequency, which may correspond to the breathing rate frequency. For example, the breathing rate frequency may be 0.75 Hz, which would correspond to 45 breaths per 60 seconds, or 45 breaths per minute, which is a breathing rate within a usual range for a newborn baby (usually ranging from 30 to 60 breaths per minute). The maximum amplitude is depicted to correspond to a value of 45 on the x-axis, or 45 breaths per minute.

The frequency spectrum 200-1 is also shown with a second amplitude peak 210-2 at a second frequency that is twice the frequency of the breathing rate frequency. It can be seen that the second frequency corresponds to 90 breaths per minute (1.5 Hz). As such, the second amplitude peak 210-2 may also be referred to as the first harmonic of the breathing rate frequency. Furthermore, the frequency spectrum 200-1 is shown with a third amplitude peak 210-3 at a third frequency that is three times the frequency of the breathing rate frequency. It can be seen that the third frequency corresponds to 135 breaths per minute (2.25 Hz). As such, the third amplitude peak 210-3 may also be referred to as the second harmonic of the breathing rate frequency. The use of the term "amplitude peak" refers to a local maximum that is significantly greater than other possible local maxima, so as to distinguish itself from other local maxima that are not significantly greater in amplitude than various local minima of the same spectrum.

Additionally, the frequency spectrum 200-1 comprises a fourth peak 212 that corresponds to a fourth frequency that is slightly below the third introduced frequency. The fourth peak may correspond to a heart rate. Within the depicted scale, the fourth peak may correspond to a heart rate that is approximately 125 beats per minute (approx. 2.08 Hz), slightly below the second harmonic frequency of 135 breaths per minute (2.25 Hz). The heart rate of 125 beats per minute is a heart rate within a usual range for a resting heart rate of a newborn baby (usually ranging from 100 to 160 beats per minute).

**Fig. 2B** depicts an exemplary averaged-frequency-spectrum 200-2. As previously described, the averaged-frequency-spectrum 200-2 is based on the breathing rate frequency. The time-domain data 130 may be divided into a plurality of sections with a common time interval that is defined by the breathing rate frequency. For example, the common time interval may be the inverse of the breathing rate frequency, such as the previously mentioned breathing rate frequency of 45 breaths per minute or 0.75 Hz, as depicted in Fig. 2A. For this example, the time interval may be a time of approximately 1.33 seconds. More specifically, the time-domain data 130 may be divided into a plurality of non-overlapping sections, with each section comprising time-domain data 130 that includes data recorded within successive time intervals, each of which may be equivalent to a time amount of 1.33 seconds. Each of the sections corresponding to a portion of the time-domain data 130 within a respective window of 1.33 seconds may then have a respective section-frequency-spectrum determined.

An average of the plurality of the section-frequency-spectra may be calculated as previously described to obtain the averaged-frequency-spectrum 200-2, which is depicted in Fig. 2B. The averaged-frequency-spectrum 200-2 also comprises a first amplitude peak 220-1 analogous to the first amplitude peak 210-1 of the (original) frequency spectrum 200-1, with analogous characteristics. For example, the first amplitude peak 220-1 is also centered at the breathing rate frequency, which is also approximately 45 breaths per minute (0.75 Hz). The averaged-frequency-spectrum 200-2 also comprises a second amplitude peak 220-2 analogous to the second amplitude peak 210-2 of the (original) frequency spectrum 200-1, with similar analogous characteristics. As depicted, this frequency is also located at a first harmonic of the breathing rate frequency. The same is depicted for a third amplitude peak 220-3 analogous to the third amplitude peak 210-3 of the (original) frequency spectrum 200-1, also located at a second harmonic of the breathing rate frequency.

The major difference between the depicted (original) frequency spectrum 200-1 and the averaged-frequency-spectrum 200-2 is the lack of a heart rate component. Rather the particular frequency component in the averaged-frequency-spectrum 200-2 near the heart rate frequency of 125 beats per minute (approx. 2.08 Hz) only comprises noise. In the averaged-frequency-spectrum 200-2, this frequency is depicted with an arrow and label showing that the "heart rate component has been filtered". The heart rate may be effectively filtered or at least mostly filtered when choosing a time interval for each of the section-frequency-spectra that corresponds to the breathing rate of the target, i.e. the inverse of the breathing rate frequency.

In this scenario, each section of the time-domain data 130 will have amplitude values corresponding to a breathing in and breathing out of the target at an analogous position of the respective section. As such, the breathing rate frequency will be depicted very similarly in the averaged-frequency spectrum 200-2 compared to the (original) frequency spectrum 200-1. Furthermore, the harmonic components appear mostly unaffected. When dividing the time-domain data 130 into sections corresponding to the inverse of the fundamental frequency, each section captures a complete period of the signal. In other words, by dividing the time-domain data 130 according to the fundamental frequency, each section aligns with a specific phase of the fundamental signal. As a result, the influence by the harmonic components, being integer multiples of the fundamental frequency, tend to remain consistent within each section of the time-domain data 130, assuming the harmonics are stable.

However, the heart rate component, unlike the first and second harmonics, is located at a position in the (original) frequency spectrum 200-1, such that its corresponding data in the plurality of sections of the time-domain data 130 does not remain aligned across the sections. Depending on its position and strength, it may be fully averaged out to no longer be visible in the averaged-frequency-spectrum 200-2 or may be mostly averaged out to be only partially visible in the averaged-frequency-spectrum 200-2. In either case, the difference spectrum may comprise a maximum amplitude that corresponds to a heart rate estimate.

While the previously described techniques including the determination of the difference spectrum and the heart rate estimation based on the maximum amplitude thereof may provide an adequately accurate heart rate estimation, this estimation may be improved by further processing techniques of the time-domain data 130. Such techniques will be discussed in greater depth with reference to Figs. 3 to 8.

**Fig. 3** provides a flow chart of exemplary pre-processing steps that may be applied before determining the frequency spectrum 200-1. The pre-processing steps includes five possible stages, or more specifically, five possible checks, which may be used in different combinations according to various embodiments. In any case where a check, such as meeting a required threshold or range, is not fulfilled, the processing circuitry 110 may be configured to keep recording (measuring) further frames of the time-domain data 130, as depicted by the flow chart step 350 to wait for the next frame. If each of the applicable checks are fulfilled, the processing circuitry 110 may be configured to proceed to further processing beyond pre-processing (i.e. to core processing), as depicted by the flow chart step 360. A first check may be a frame number check 310-1. For example, the processing circuitry 110 may be automated to accept a certain number of frames of the time-domain data 130 up to a pre-specified frame-threshold 310-2. The frame-threshold 310-2 may be 500 frames or another pre-specified number.

The pre-specified number of frames may correspond to a pre-specified amount of time (i.e. a time window) that is designated for receiving the receive signal. More specifically, the time-domain data 130 may correspond to the time window. For the example of an FMCW radar sensor, if the time window is 10 seconds, the time-domain data 130 may correspond to 10 seconds of the radar system emitting frequency-modulated continuous wave signals and receiving the corresponding echoed (i.e. reflected) signals. For example, in such a case, the apparatus 100 may receive 500 frames in 10 seconds, or 1 frame every 0.02 seconds. The time window of 10 seconds may be chosen for a desired balance. On one hand, it may be beneficial to obtain more time-domain data for more accurate averaging. On the other hand, the time window should not be extended too long, where a random body movement of the target or other possible disturbances have a higher likelihood to cause the time-domain data 130 to become invalid. For example, in the case of the target being a baby, it becomes increasingly unlikely after 10 seconds that the baby will remain adequately still for obtaining accurate measurements for valid time-domain data 130. The time window may be adjusted according to each use case or a user input, such as ranging between 8 and 12 seconds.

A second check may be a noise check 320-1. The noise check 320-1 may be used to determine whether the time-domain data 130 based on the receive signal is below a pre-specified noise-level threshold 320-2. For example, the processing circuitry 110 may be configured to apply a high-pass filter for a processing of the time-domain data 130. Based on the high-pass filter processing, the processing circuitry 110 may be configured to calculate an average amplitude of the high-pass filtered time-domain data 130 to obtain a noise-level average. The noise-level average may then be checked if it is below the noise-level threshold 320-2. Proceeding forward, the processing circuitry 110 may be configured to determine the frequency spectrum 200-1 if the noise-level average is under a pre-specified noise-level threshold 320-2.

A third check may be a range check 330-1. The range check 330-1 may be used to determine whether the receive signal comprises sufficient time-domain data from a pre-specified valid range 330-2. If it is determined that the time-domain data 130 or a required amount of the time-domain data 130 does not correspond to the pre-specified range, the processing circuitry 110 may be configured to record new measurements for new time-domain data 130 (i.e. waiting for the next frame 350 for new measurements). The range of the time-domain data 130 may be determined by a range discrete Fourier transformation (DFT) of the time-domain data 130. The range-DFT may lead to the apparatus 100 obtaining a range buffer divided into a plurality of range bins. More specifically, the results of the range-DFT may be stored in a memory of the apparatus 100, wherein portions of the time-domain data 130 are stored according to the respective range bins. A first range bin may correspond to a first range from the radar sensor, a second range bin may correspond to a second range from the radar sensor, etc.

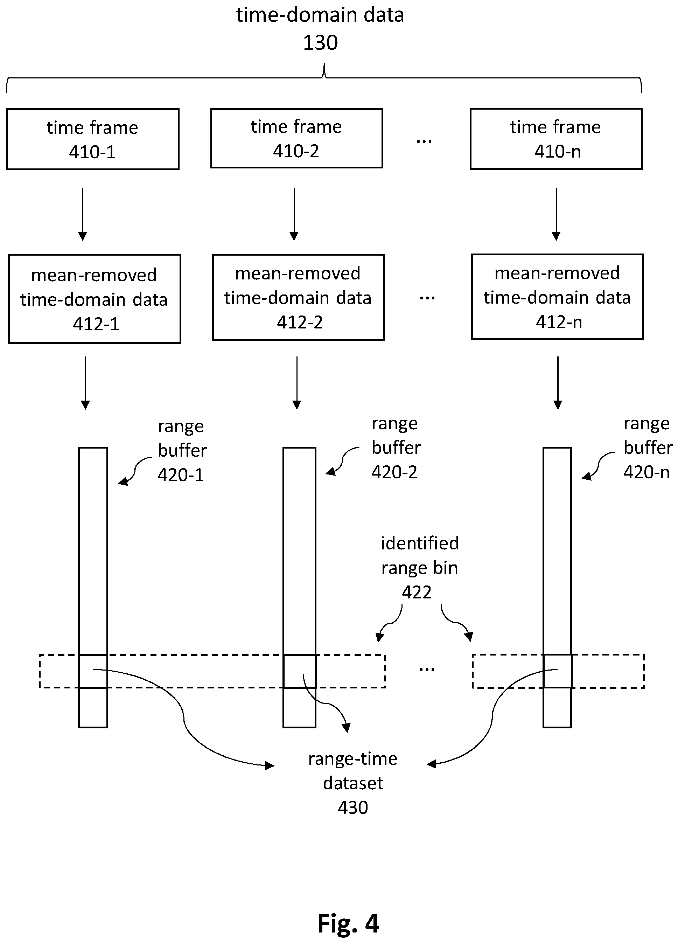

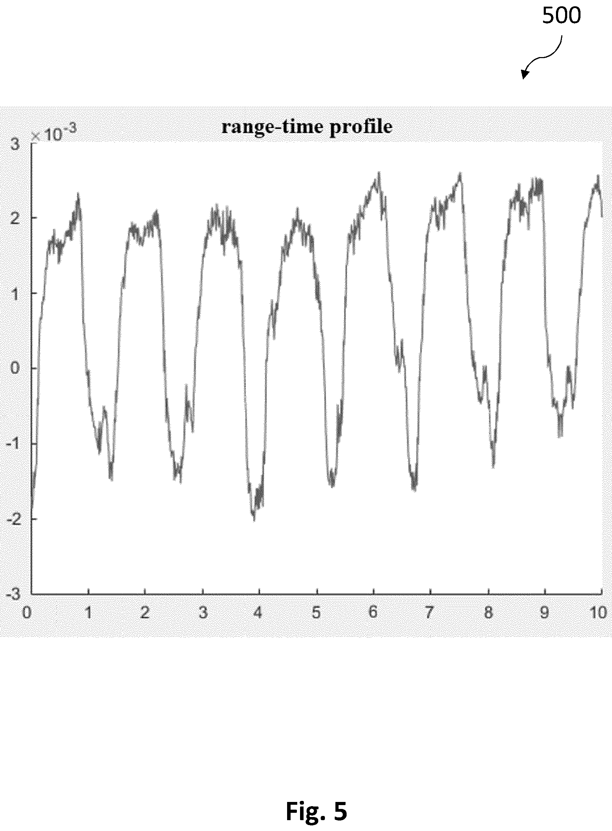

As part of or in addition to the range check 330-1, the range-DFT may also be performed on the basis of a plurality of frames of the time-domain data 130, which will be discussed in greater detail with reference to Fig. 4. Furthermore, two breathing checks 340-1a; 340-1b (i.e. breathing pattern checks) based on respective breathing check verifications 340-2a; 340-2b, will be explained with reference to Fig. 5.

**Fig. 4** provides a visual representation for exemplary processing steps for measuring a heart rate of a target in a field of view of a radar sensor, including the above-described range-DFT. As depicted, the processing circuitry 110 may obtain a plurality of frames 410-1; 410-2; 410-n, which are maintained as separate units of the time domain data 130. The three dots between the 2^{nd} frame and n-th frame are meant to express any number of frames that may be part of the time-domain data 130 between the 2^{nd} frame and n-th frame. As previously described, the number of frames may be 500 or another pre-specified number.

For example, the processing circuitry 110 may be configured to perform mean removal of the time-domain data 130. More specifically, the processing circuitry 110 may be configured to calculate a mean value of amplitude values of the time-domain data 130 and to subtract the mean value from the amplitude values to generate mean-removed time-domain data. This may shift the previous values, which may include an offset, to be centered on zero. Generally, using a form of mean-removed time-domain data 130 may enable generating the frequency spectra 200-1; 200-2 more accurately. If applicable, the mean-removed version of the time-domain data 130 may also be used to perform further checks more accurately, such as confirming a valid breathing rate and heart rate for the target (provided in Fig. 5).

The mean removal may be applied for the entire time-domain data 130 or on a per frame basis. Using a per frame basis, the mean value of amplitude values may be a mean value of the given frame, which may be subtracted from the amplitude values of the given frame. This may generate mean-removed time domain data specifically for the given frame. As depicted, a first portion of mean-removed time-domain data 412-1 may correspond to a first time frame 410-1, a second portion of mean-removed time-domain data 412-2 may correspond to a second time frame 410-2, up to an n-th portion 412-n for an n-th frame 410-n. Furthermore, the previously described range-DFT may be performed using the various portions of mean-removed time-domain data. Further references to the processing of the time-domain data 130 may apply either to mean-removed or non-mean-removed time domain data 130.

The processing circuitry 110 may be configured to perform a separate range-DFT for each frame 410-1; 410-2; 410-n (i.e. on a per frame basis) of the time-domain data 130.. If mean-removal of the time-domain data is applied, the range-DFT may be performed for each portion of the mean-removed time-domain data 412-1; 412-2; 412-n corresponding to their respective frames. As such, the range-DFT may be referred to as a short time range-DFT being applied to short time range data (respective frames or portions) of the time-domain data 130. Each of the respective range-DFTs being performed on the respective frames or portions of the (mean-removed) time-domain data 130 may lead to generating a respective range buffer 420-1; 420-2; 420-n, as depicted, corresponding to each frame in Fig. 4. In the case that the number of frames for a full iteration of measurements is pre-specified to be 500 frames (i.e. n = 500), the processing circuitry 110 may perform 500 respective range-DFTs for 500 frames or portions of the (mean-removed) time-domain data 130 to obtain 500 respective buffers. Each of the range buffers may comprise an equal number of range bins, so that a particular range bin of a first range buffer may have a corresponding range bin at an equivalent position or with an equivalent index within a second range buffer.

The processing circuitry 110 may identify a particular range bin for each range buffer with the greatest value (e.g., greatest amplitude value or mean-removed amplitude value) of the (mean-removed) time-domain data 130. More specifically, a collection of corresponding range bins across the plurality of (e.g., 500) range buffers may be chosen, wherein the collection of range bins each correspond to an equivalent index or position within its respective range buffer. The processing circuitry 110 may be configured to choose such a collection of range bins corresponding to a common position or index, each (or most) of the range bins comprising a maximum amplitude of the (mean-removed) time-domain data 130. As such, the collection of corresponding (i.e. analogously positioned) range bins may also be referred to simply as a collective "range bin" or "identified range bin", referring specifically to the common position within each range buffer. Fig. 4 shows an identified range bin 422 corresponding to a maximum amplitude for the (mean-removed) time-domain data 130.

The processing circuitry 110 may be configured to check whether or not the identified range bin 422 is within a pre-specified selection of range bins for the respective range buffers. The pre-specified selection of range bins may correspond to a range of measurement within an acceptable range of distance away from the radar sensor. Such an acceptable range of distance may be, for example, between 1 to 1.5 meters, or another acceptable range of a similar scale (e.g., between 0.5 meters and 3 meters). If the identified range bin 422 is not within the pre-specified selection of range bins, the time-domain data 130 may be considered to be invalid and a new iteration of measurements by the radar system may begin (i.e. wait for the next frame 350). On the other hand, if the identified range bin 422 is indeed within the pre-specified selection of range bins, the time-domain data 130 may be considered to be within a pre-specified valid range 330-2.

Based on a determination of a valid range of the time-domain data 130, the processing circuitry 110 may determine the frequency spectrum 200-1 and the averaged-frequency-spectrum 200-2 for the (mean-removed) time-domain data 130 corresponding to the identified range bin 422 for each range buffer 420-1; 420-32; 420-n. In other words, the processing circuitry 110 may be configured to transform the (mean-removed) time-domain data 130 by a range-DFT, select a portion of the data generated, and determine the frequency spectrum 200-1 based on the selected data, which corresponds to a certain portion of the time-domain data 130.

Since the range-DFT may be applied across the range buffers, it may be considered as being applied to long time-domain data. By focusing the Doppler-DFT to the identified range bin 422, the frequency spectra 200-1; 200-2 may be more accurately determined.

The processing steps presented in Fig. 4, including the range-DFT applied to the (mean-removed) time-domain data, may generate a (mean-removed) range-time dataset 430. The range-time dataset 430 is depicted to include the data within the identified range bin 422. Based on the identified range bin 422, the range-time dataset 430 may be extracted for further checks 340-1a; 340-1b.

**Fig. 5** depicts an exemplary range-time profile 500 corresponding to the range-time dataset 430. The range-time profile 500 may be used for checking if there is a consistent pattern (i.e. breathing pattern) that corresponds to a breathing of the target. Such breathing pattern checks 340-1a; 340-1b may be performed before the Doppler-DFT, wherein the Doppler-DFT is only performed if the breathing pattern checks 340-1a; 340-1b pass respective thresholds 340-2a; 340-2b (see Fig. 3).

The breathing pattern checks 340-1a; 340-1b, which may be applied to the time-domain data 130 or specifically, the mean-removed time-domain data, as previously described, corresponding to the identified range bin 422. Alternatively, the processing circuitry 110 may be configured to perform mean removal after the range-DFT, for which a mean value of amplitude values in the identified range bin 422 is calculated and subtracted from the mean value from the amplitude values in the identified range bin 422. This may shift the information of the time-domain data 130 corresponding to the identified range bin 422 from the previous values to zero-centered values to generate the mean-removed range-time dataset 430 for the identified range bin 422. The mean-removed range-time dataset 430 may enable faster processing for further checks of the data to ensure that a valid breathing rate and heart rate may be found for the target in the field of view of the radar sensor. If such checks are passed, then the frequency spectrum may be determined specifically based on the mean-removed range-time dataset 430.

The exemplary range-time profile 500 based on the mean-removed range-time dataset 430 (with mean-removal performed before or after the range-DFT) shows visually how this shifts the time-domain data 130 to be centered around zero. The x-axis of the range-time profile 500 may be correspond to any arbitrary unit of time (e.g., seconds), while the y-axis of the range-time profile 500 may correspond to a complex number amplitude directly proportional to a range (i.e. distance or displacement), which may be measured using an appropriate unit of thereof.

In fact, the range-time profile 500 may provide a visual depiction of a breathing pattern when plotted. A breathing of the target may include a breathing in and a breathing out, which may repeat in a steady pattern over time. For example, while breathing in, air is inhaled and a chest of the target may expand, which may thus decrease the range (i.e. distance or displacement) between the target and the radar sensor. On the other hand, while breathing out, air is exhaled and the chest of the target may contract, which may thus increase the range (distance or displacement) between the target and the radar sensor. Since a breathing pattern must follow a recognizable pattern, certain checks may be implemented to ensure a proper breathing of the target. With a consistent breathing, the processing steps outlined in Figs. 1 and 2 may be reliably performed to estimate the heart rate.

The mean-removed range-time dataset 430 may correspond to the mean-removed range-time profile 500, which may depict a variation in the range (distance or displacement) that may be checked for consistency. In the first breathing check 340-1a presented in Fig. 3, the processing circuitry 110 may be configured to check whether or not a peak-to-peak value for the mean-removed range-time dataset 430 is within pre-specified limits based on an average of a plurality of previous peak-to-peak values in the mean-removed range-time dataset 430.

For example, a first peak-to-peak value corresponding to an increase in range (distance or displacement) starting at a time *t =* 0 may be approximately 4.0 x 10⁻³ arbitrary units,. A second peak-to-peak value corresponding to a decrease in range (distance or displacement) may be approximately 3.5 x 10⁻³ units. A third, fourth, and fifth peak-to-peak values may also approximately 3.5 x 10⁻³ units, while a sixth peak-to-peak value, for the third decrease in range (distance or displacement), may be approximately 4 x 10⁻³ units. A plurality of peak-to-peak values may be checked. Optionally, each peak-to-peak value may be checked, wherein a peak-to-peak is determined to correspond to any increase from a local minimum to a local maximum or to correspond to any decrease from a local maximum to a local minimum (wherein local minima or local maxima of a very narrow frequency range may be neglected). A peak-to-peak-threshold 340-2a (depicted as part of the breathing check verification in Fig. 3) for all peak-to-peak values may be determined based on a plurality or all of the peak-to-peak values, such as by averaging. The processing circuitry 110 may be configured to determine the frequency spectrum for the mean-removed range-time dataset 430 if all or a large majority of the peak-to-peak values, as previously presented, are within the limits of the peak-to-peak-threshold 340-2a.

In a second breathing check 340-1b in Fig. 3, the processing circuitry 110 may be configured to determine a plurality of derivative values corresponding to a rate of change of a range at a respective time of the mean-removed range-time dataset 430 (or the range time profile 500). Each derivative may be determined based on an instance of time, which may be within or correspond to an interval of time. The x-axis of the range-time profile 500 may correspond to a time unit of seconds. As such, the range-time profile 500 may correspond to the previously mentioned time window of 10 seconds. Each full cycle from a local minimum (or maximum) to a neighboring local minimum (or maximum) may thus correspond to a time of a 1.33 seconds for one complete breath, which is a time interval that corresponds to a breathing rate frequency of 0.75 Hz, or a breathing rate of 45 beats per minute. Within the time window of 10 seconds, 8 inhalations (increases in range/displacement) are depicted, along with 7 exhalations, corresponding to approximately 7.5 breaths for the 10 seconds (0.75 Hz).

Each inhalation may have associated therewith a derivative value. For example, a derivative value may correspond to change in units of range (distance or displacement) of the dataset from the local minimum to the local maximum divided by the change in time in seconds. The same may analogously be true for a change from a local maximum to a local minimum (wherein local minima or local maxima of a very narrow frequency range may be neglected, as previously described). Alternatively or additionally, a derivative value may correspond to a change in range (displacement) divided by a corresponding change in time, wherein the time interval within the range-time profile 500 for the corresponding range and time changes may be arbitrarily chosen. The processing circuitry 110 may be configured to determine any number of derivative values within the range-time profile 500, which may be from a local minimum to a local maximum or vice versa, or which may correspond to a narrower time interval within such points.

The processing circuitry 110 may be configured to determine a variance of such derivative values as a derivative-threshold 340-2b (depicted as part of the breathing check verification in Fig. 3), which may be based on an absolute value of the determined derivatives. Furthermore, the processing circuitry 110 may check that all or a large majority of the determined derivatives (or their absolute values) are within the derivative-threshold 340-2b. The processing circuitry 110 may then determine the frequency spectrum 200-1 for the mean-removed range-time dataset 430 if each derivative value is within the derivative-threshold 340-2b.

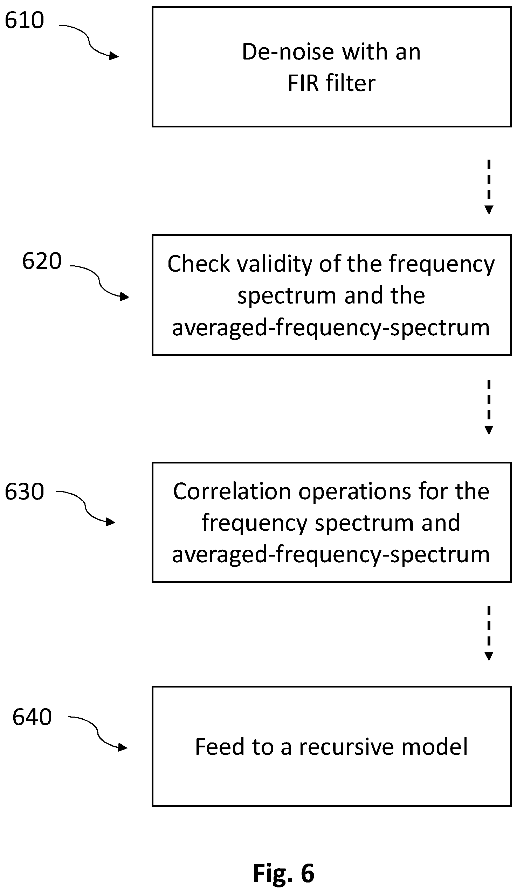

Generally, the five above-described checks 310-1; 320-1; 330-1; 340-1a; 340-1b may be used in any combination to ensure by means of their respective thresholds 310-2; 320-2; 330-2; 340-2a; 340-2b that the time-domain data 130 is valid data, which may be considered reliable for determining a breathing rate and a heart rate of the target within the field of view of the radar sensor. Such checks may be part of a pre-processing of the apparatus 100. If all applied checks are passed, the processing by the apparatus 100 may continue to its core-processing steps, as outlined in Figs. 1 and 2. In addition to the previously outlined core-processing steps, the core-processing may include further processing features, which will be presented in greater detail with reference to Fig. 6.

**Fig. 6** provides a flow chart of exemplary optional processing steps 610; 620; 630; 640 that may be applied for measuring a heart rate of a target in a field of view of a radar sensor.

A first optional processing step shown in Fig. 6 is de-noising 610 with a finite-impulse-response filter. The processing circuitry 110 may be configured to determine the frequency spectrum for the time-domain data and to subsequently apply a finite-impulse-response filter to the amplitude values of the frequency spectrum. A finite-impulse-response, FIR, filter is generally a digital filter used in signal processing. The FIR filter may be used, particularly as a specific form of a high-pass filter, to eliminate signals corresponding to vibrating objects that may be present in a background of the target in the field of view of the radar sensor. The vibrating may include a switching back and forth between positive and negative directions of movement through space. In other words, the vibrating may be switching back and forth between having a positive velocity in a positive direction and a negative velocity in a negative direction, which may be recorded as part of the time-domain data 130 and may disturb the signal processing of the time-domain data 130. For example, air conditioning is well-known as a vibrating object that may interfere with signal processing techniques of radar systems, among other possible causes.

Whereas FIR filters may conventionally or usually be used for filtering (i.e. filtering by data processing) of time-domain data before a Doppler-DFT, the processing circuitry 110 may be specifically configured to apply the FIR filter after the Doppler-DFT to data of the generated frequency spectrum 200-1 (i.e. the time-domain data 130 in processed form as part of the frequency spectrum 200-1) to prevent a disturbance in signal processing by a vibrating object. For example, for a wave signal emitted by a frequency-modulated continuous wave, FMCW, radar, a frequency component in a Doppler-DFT analysis may correspond to a speed or velocity of an object in the field of view of the radar sensor, as derived from the Doppler Effect, describing how the frequency of an emitted and reflected wave may change when reflected off an object with a relative motion from the radar sensor.

While an FIR filter, such as an FIR-high-pass filter, may filter data before a Doppler-DFT in the time-domain according to a high speed or a particular speed component within the signal, an FIR filter applied to filter the data after a Doppler-DFT in the frequency domain may filter a high change of speed, or a particular change-of-speed component within the data. As such, a disturbance in the data by a vibration, which may correspond to a particular change of speed that causes movement in the previously described positive and negative directions, may be filtered. In general, various versions of the FIR filter may be applied to filter the influence of various vibrating objects in the field of view of the radar sensor, particular for vibration ranges of common household objects, such as an air-conditioning unit.

A second optional processing step shown in Fig. 6 relates to check the validity 620 of the frequency spectrum 200-1 and the averaged-frequency-spectrum 200-2. In a first check, the processing circuitry 110 may be configured to determine the breathing rate frequency by detection of a first frequency corresponding to a maximum amplitude value of the frequency spectrum 200-1 and detection of a second frequency corresponding to a local maximum of amplitude values in the frequency spectrum 200-1 within a pre-specified frequency range from twice the first frequency (e.g. a first harmonic). Referring now back to Fig. 2 and the depicted (original) frequency spectrum 200-1, the breathing rate frequency may be detected at its maximum amplitude 210-1, corresponding to the breathing rate (e.g., 45 breaths per minute), while the following local maximum may be detected within a pre-specified range of twice the breathing rate frequency (e.g., 90 breaths per minute). In this particular case, the pre-specified frequency range for the following local maximum may be between 87 and 93 breaths per minute, or another frequency range based on accuracy or precision requirements, which may be adjustable.

Furthermore, the processing circuitry 110 may be configured to detect a third frequency corresponding to another local maximum of amplitude values in the frequency spectrum, specifically within a second pre-specified frequency range from three times the first frequency (i.e. second harmonic). In the (original) frequency spectrum 200-1 of Fig. 2, the second following local maximum from the breathing rate frequency may be detected at 135 breaths per minute or within the second pre-specified range therefrom. In this particular case, the second pre-specified range may be 132 to 138 breathes per minute, or another frequency range that may also be adjustable based on accuracy or precision requirements. Particularly for the second harmonic of the breathing rate frequency, the pre-specified range may be narrow enough to distinguish the second harmonic from the local maximum corresponding to the heart rate frequency. If either the frequency values of the first or second harmonics are not within the respective pre-specified ranges, the processing circuitry 110 may be configured to label the time-domain data 130 as invalid and cancel further processing of the time-domain data 130 and begin the procedure for recording a new iteration of time-domain data.

Such validity tests may also be performed related to the averaged-frequency-spectrum 200-2 in Fig. 2. For example, the processing circuitry 110 may be configured to verify that a maximum amplitude value of the averaged-frequency-spectrum 200-2 corresponds to a frequency that is within a pre-specified frequency range from the breathing rate frequency. Such a check may provide an accurate assessment of whether or not the breathing rate frequency that is determined based on the maximum amplitude of the (original) frequency spectrum 200-1 is accurate. In a case where the breathing rate frequency is not accurate, the time-domain data 130 may not have been divided into its plurality of sections with an adequately accurate time interval, being the inverse of a somewhat inaccurate breathing rate frequency. This may have led to a shift in the maximum-amplitude frequency to a frequency in the averaged-frequency-spectrum 200-2 away from the breathing rate frequency in the (original) frequency spectrum 200-1. For example, such a pre-specified range may be between 42 and 48 breaths per minute, or it may adjustable to another range, depending on accuracy and precision requirements. If the maximum amplitude value of the averaged-frequency-spectrum 200-2 corresponds to a frequency that is within its pre-specified frequency range from the breathing rate frequency, then the later processing steps towards determining the difference spectrum and estimating the heart rate may be performed.

If this is not the case, the processing circuitry 110 may be configured to determine a new breathing rate frequency based on a neighboring local maximum of amplitude values in the (original) frequency spectrum 200-1. For example, a highly localized spike may be present in the determined (original) frequency spectrum 200-1, which may have caused the assignment of a somewhat inaccurate value for the breathing rate frequency. To prevent this, the processing circuitry 100 may be configured to accept a breathing rate frequency that corresponds to an amplitude within a certain pre-specified range. If the breathing rate frequency is determined to be inaccurate (for any reason), the processing circuitry 110 may be configured to repeat its previously performed calculations for a following or previous local maximum from the previously labeled breathing rate frequency (wherein local minima or local maxima of a very narrow frequency range may be neglected, as previously described).

Such a local maximum may also be searched for within a customized frequency range that may correspond to a breathing rate that corresponds to the physical characteristics of the target. For example, a target that is a human newborn infant may have a known range for breathing rate frequency, given certain assumptions (e.g., being at rest). A newborn infant may have such a search range vary between 20 and 70 breaths per minute, an older baby/toddler between 15 and 40 breaths per minute, and an adult between 8 and 25 breaths per minute. Other physical characteristics besides the age of the target may also affect the customized range. Such configurations may be adjustable. Adjustable configurations may be adjusted by a user input received through a user interface, among other possible means.

The processing circuitry 110 may be configured to repeat the same processing steps for the new breathing rate frequency, such as for a following or previous local maximum, to obtain a new averaged-frequency-spectrum based on the new breathing rate frequency. Furthermore, the previously described checks for the maximum amplitude value of the new averaged-frequency-spectrum may be verified to correspond to a frequency that is within its pre-specified frequency range from the new breathing rate frequency. If this is not the case, another local maximum of the (original) frequency spectrum 200-1 may be checked if it is within the customized frequency range based on the target's physical characteristics, with the previously described steps repeated. If no local maximum within the customized frequency range meet the required checks, then the processing circuitry 110 may be configured to label the time-domain data 130 as invalid and to cancel further processing thereof, as well as to begin the procedure for recording a new iteration of time-domain data.

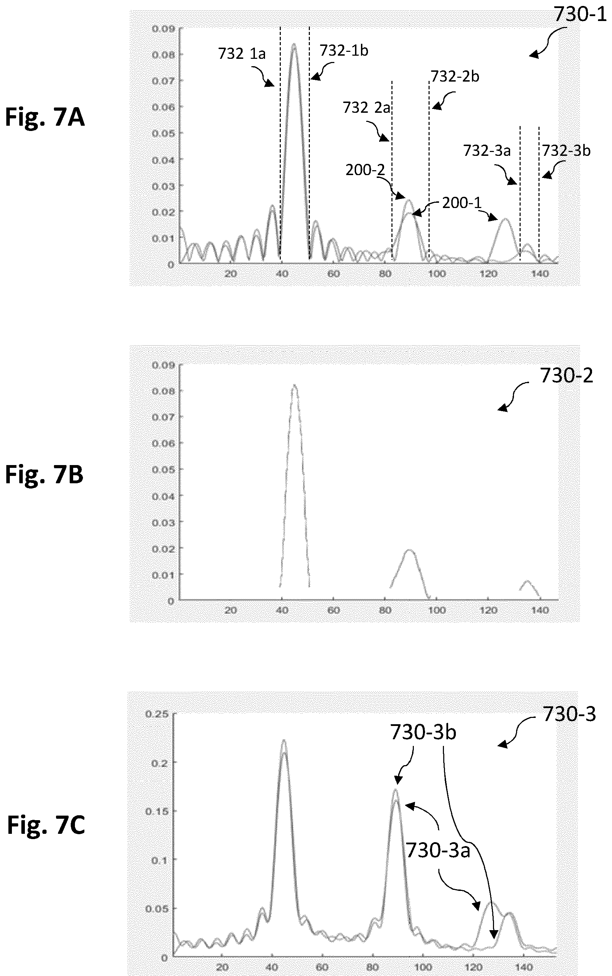

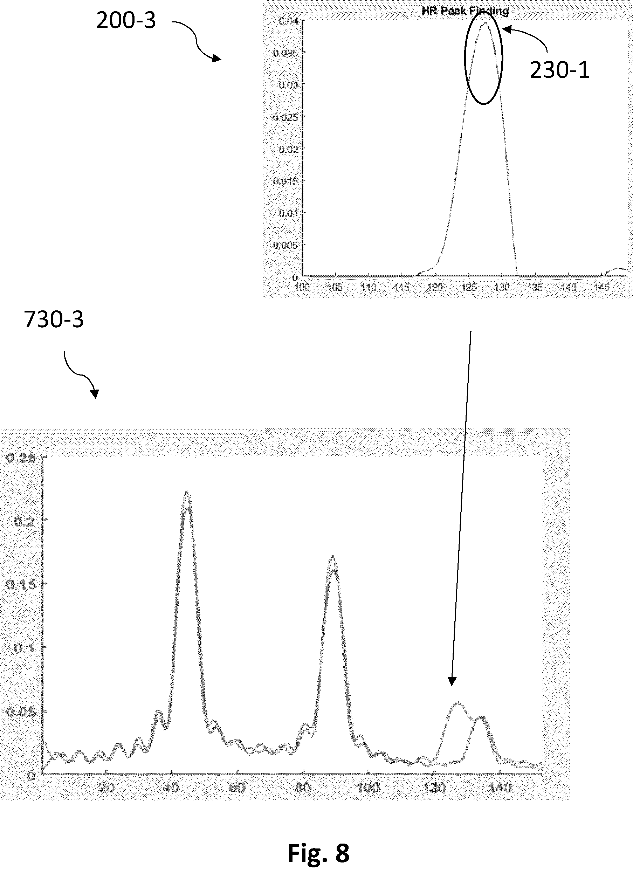

While multiple examples related to calculation thresholds have been explained in depth, the processing circuitry 110 may be configured to apply a variety of thresholds for ensuring that an accurate value of the breathing rate frequency and heart rate frequency is determined/estimated. Such thresholds may be applied in various combinations and at various stages of processing. As depicted in Fig. 6, further examples of processing of the time-domain data 130 to improve accuracy and/or precision of the heart rate estimation, and possibly the breathing rate estimation, is the application of correlation functions 630 (to be discussed with reference to Figs. 7A, 7B, and 7C) and using a recursive model of heart rate estimations 640 (to be discussed with reference to Fig. 8).

**Fig. 7A** depicts an exemplary (original) frequency spectrum 200-1 (with a heart rate component) and an exemplary averaged-frequency-spectrum 200-2 (without a heart rate component) plotted together. They are each provided in the context of an exemplary non-correlated spectrum 730-1. If an accurate value for the breathing rate frequency is selected, then the maximum amplitudes of the two spectra, as well the local maxima of the respective first and second harmonics, highly overlap, as shown in the non-correlated spectrum 730-1. However, in order to be able to further increase the accuracy and/or precision of the heart rate estimation, both the (original) frequency spectrum 200-1 and the averaged-frequency-spectrum 200-2 may undergo a correlation operation.

More specifically, the processing circuitry 110 may be configured to perform a first correlation operation for the (original) frequency spectrum 200-1 to obtain a correlated (original) frequency spectrum (in Fig. 7C as 730-3a) and to perform a second correlation operation for the averaged-frequency-spectrum 200-2 (in Fig. 7C as 730-3b) to obtain a correlated averaged-frequency-spectrum (in Fig. 7C as 730-3). Each correlation operation may be between an original version and a windowed version of the respective spectrum, wherein the respective windowed version is altered by applying a first Gaussian window centered on a first frequency corresponding to a maximum amplitude of the spectrum, applying a second Gaussian window centered on a second frequency that is two times the first frequency, applying a third Gaussian window centered on a third frequency that is three times the first frequency, and nullifying all amplitude values outside of the three Gaussian windows.

For example, in Fig. 7A, the (original) frequency spectrum 200-1 is depicted with its first, second, and third Gaussian windows. The first Gaussian window for the (original) frequency spectrum 200-1 is determined corresponding to the depicted vertical lines 732-1a and 732-1b. Each are located at a frequency corresponding to its immediately previous and immediately following local minima from the maximum amplitude, respectively. The second Gaussian window for the (original) frequency spectrum 200-1 is provided by the depicted vertical lines 732-2a and 732-2b, also by its immediately previous and immediately following local minima from the second highest local maximum within the (original) frequency spectrum 200-1. The third Gaussian window for the (original) frequency spectrum 200-1 is provided by the depicted vertical lines 732-3a and 732-3b and are determined in an analogous fashion.

The first, second, and/or third Gaussian windows may also be determined by a different means, such as by a pre-specified frequency range from the respective maximum, which may be customized. While respective first, second, and third Gaussian windows of the averaged-frequency-spectrum 200-2 may also be used in its correlation operation, they are not depicted for the sake of clarity in Fig. 7A.

Once the first, second, and third Gaussian windows have been determined, they may be applied to generate a windowed (original) frequency spectrum 730-2, depicted in Fig. 7B. The first correlation operation may be between the (original) frequency spectrum 200-1 and the windowed (original) frequency spectrum 730-2. Generally, in a correlation operation, two data points from the two frequency spectra being correlated, respectively, may be multiplied and added to a recursive sum in a first correlation iteration of the correlation operation. This process may be repeated for a second iteration of the correlation operation, wherein one of the spectra is shifted with respect to the other spectrum to determine new sets of data points to be correlated, with new multiplication products determined and added to the recursive sum. Such iterations may be repeated according to further equivalent shifts to determine a first correlation spectrum 730-3a, which may depict a result of the first correlation operation, performed between the (original) frequency spectrum 200-1 and the windowed (original) frequency spectrum 730-2.

Furthermore, the above-described techniques may be repeated for the previously described second correlation operation. The second correlation operation may be between the averaged-frequency spectrum 200-2 and a windowed version of the averaged-frequency-spectrum (not depicted, analogous to 730-2) that is analogous to the windowed version of the (original) frequency spectrum 200-1. In other words, the averaged-frequency spectrum may also have a first, second, and third Gaussian window applied (not depicted, analogous to 732-1a; 732-1b; 732-2a; 732-2b; 732-3a; 732-3b), as previously described, to form its windowed version. The correlation operation may then be performed, also as previously described, to obtain a respective correlation spectrum 730-3b.

**Fig. 7C** depicts an exemplary correlated spectrum 730-3, including the exemplary correlated frequency spectrum 730-3a and the exemplary correlated averaged-frequency-spectrum 730-3b after respective correlation operations. Once the two correlated frequency spectra 730-3a; 730-3b are determined, they may comprise greater similarities within the windowed portions, while portions of the frequency spectra 730-3a; 730-3b outside of the windowed portions may be suppressed toward lower amplitude values, as can be seen with a comparison between the non-correlated spectra of 730-1 in Fig. 7A and the correlated spectra of 730-3 in Fig. 7C. The result of the correlated spectrum 730-3 shows that the correlated (original) frequency spectrum 730-3a and the correlated averaged-frequency-spectrum 730-3b overlap more precisely, which may enable canceling the respective breathing rate components more effectively in the difference spectrum. As such the heart rate component 212 in Fig. 2A may be determined more accurately.

**Fig. 8** provides the same exemplary correlated spectrum 730-3 in Fig. 7C, but with a difference spectrum 200-3. The difference spectrum 200-3 is determined by subtracting the averaged-frequency-spectrum 200-2 from the (original) frequency spectrum 200-1. In particular, the processing circuitry 110 may be configured to subtract the correlated averaged-frequency-spectrum 730-3b from the correlated (original) frequency spectrum 730-3a to obtain the difference spectrum 200-3, wherein the subtraction of one spectrum from another follows the previous definition provided for Fig. 1. Since the respective correlated spectra emphasize the frequency components corresponding to the breathing rate frequency, as well as its first and second harmonic components, it may enable the difference spectrum 200-3 to be determined more accurately compared to not using the correlated spectra. Furthermore, the heart rate may be estimated as corresponding to a maximum amplitude 230-1 of the difference spectrum 200-3, as depicted. The difference spectrum 200-3 is depicted with a zoomed-in scale and shows the heart rate component at a frequency of the heart rate (shown with greater precision to be approximately 128 beats per minute). This is depicted by the heart component 212 in Fig. 2A.

Additionally, the processing circuitry 110 may be configured to apply a window to the difference spectrum 200-3, and to search for the maximum amplitude 230-1 within the window of the difference spectrum 200-3 for estimation of the heart rate frequency. The window of the difference spectrum 200-3 may specify a frequency range corresponding to a range of possible heart rates of the target, which may also be customized (e.g., from a user input) according to the target in the field of view of the radar sensor.

The processing circuitry 110 may also be configured to record multiple heart rate estimations within a single recording iteration or briefly separated recording iterations. For example, given a time window of 10 seconds or a similar value (e.g., 8 to 12 seconds) for a measurement iteration, approximately 5 to 7 estimations for the heart rate of the target may be performed within one minute of time. If the target is adequately still with minimal random body movement, then all estimations of the respective measurement iterations may be determined to be valid. Alternatively, if too much random body movement has occurred in a time window, thereby preventing a measurement of valid data for the heart rate estimation, the time-domain data corresponding to that particular time window may be discarded and further measurement iterations may continue. For example, the processing circuitry 110 may be configured to stop measuring and immediately begin a new measurement iteration (e.g. of 10 seconds) if a threshold related to random body movement has been surpassed, which may include the checks and corresponding thresholds described in Fig. 3.

In particular, the processing circuitry 110 may be configured to perform multiple heart rate estimations, wherein each estimation is applied to an iteration of a recursive model for obtaining a refined heart rate estimate. For example, a first estimated value for the heart rate of the target may be based on a first heart rate estimate with a first range of error. Given a second heart rate estimate of the same target immediately following the first heart rate estimate, wherein both estimates are based on respective time-domain data 130 under the same circumstances, the second heart rate may increase confidence of the previous estimate. Furthermore, the recursive model may determine that the heart rate of the target is a value within a smaller range of error.

The recursive model may continue to be applied, wherein further, e.g., five or more, estimated values for the heart rate of the target may be given. Based on each successive estimation, the recursive model may determine an increased probability that the heart rate of the target is within a narrowed range. Generally, such a narrowed range may be part of the refined heart rate estimate, wherein the final estimate is considered to be more accurate and/or precise.

In addition to a refined heart rate estimate, the apparatus 100 may also to be used to increase confidence in a breathing rate estimate (i.e. breathing rate determination). Based on many possible checks applying the breathing rate frequency and its first and second harmonics, a higher confidence in the breathing rate may be obtained. Furthermore, other possible checks (e.g., checks related to the heart rate), which may not even be possible without an accurate value of the breathing rate, may also be done and verified, further increasing confidence in the breathing rate.

In general, the apparatus 100, used in accordance with one of the various above-described configurations may provide a particular benefit of long-term monitoring biological signs of the same target. In the particular case of a newborn baby, heart rates and breathing rates may be measured and recorded multiple times and performed over many different days (or longer time spans), which may provide caregivers with more reliable information related to biological activity and overall health of the newborn baby. Such a benefit is also possible for older children and adults. A further benefit may be to better understand patterns of such biological activity in a variety of target types (e.g., human babies or adults, or also animals) and how they may be affected, particularly if other events occurring within an associated time span are also recorded. Generally, the apparatus 100 provides a new means for examining biological activity and health by a new technique using radar systems.

**Fig. 9** illustrates an example of a radar system 900 comprising an apparatus 910 as described herein, such as apparatus 100, and a radar sensor 920, configured to generate the receive signal. The radar sensor 920 may, for instance, be an FMCW (Frequency-Modulated Continuous Wave) radar sensor.

Although the apparatus 910 and the radar sensor 920 are depicted as separate blocks in Fig. 9, in other examples, the apparatus 910 may in part or in entirety be included in the radar sensor 920. Thus, the radar sensor 920 may comprise the apparatus 910. In case the apparatus 910 is only partially included in the radar sensor 920, the radar system 900 may perform distributed processing carrying out respective parts of processing steps. In case the apparatus 910 is integrated in the radar sensor 920, the control circuitry and the radar sensor 920 may be jointly integrated (embedded) in a single semiconductor chip, or in more than one semi-conductor chip.

More details and aspects of the radar system 900 are explained in connection with the proposed technique or one or more examples described above, e.g., with reference to Fig. 1. The radar system 900 may comprise one or more additional optional features corresponding to one or more aspects of the proposed technique, or one or more examples described above.

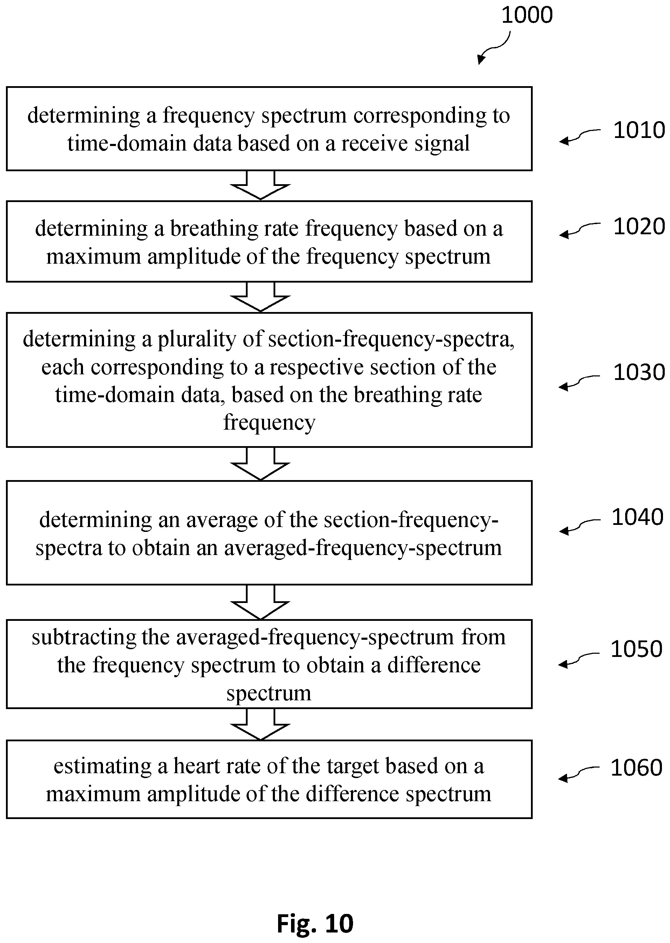

**Fig. 10** illustrates a flowchart of an example of a (e.g., computer-implemented) method 1000. The method 1000 may be performed by an apparatus described herein, such as apparatus 100. The method 1000 comprises determining a frequency spectrum 1010 corresponding to time-domain data based on a receive signal. The method 1000 further comprises determining a breathing rate frequency 1020 based on a maximum amplitude of the frequency spectrum. Furthermore, the method 1000 comprises determining a plurality of section-frequency-spectra 1030, each corresponding to a respective section of the time-domain data, based on the breathing rate frequency, and determining an average 1040 of the section-frequency-spectra to obtain an averaged-frequency-spectrum. The method further comprises subtracting 1050 the averaged-frequency-spectrum from the frequency spectrum to obtain a difference spectrum and estimating 1060 a heart rate of the target based on a maximum amplitude of the difference spectrum.

More details and aspects of the method 1000 are explained in connection with the proposed technique or one or more examples described above, e.g., with reference to Fig. 1. The method 1000 may comprise one or more additional optional features corresponding to one or more aspects of the proposed technique, or one or more examples described above.

Examples may further be or relate to a (computer) program including a program code to execute one or more of the above methods when the program is executed on a computer, processor or other programmable hardware component. Thus, steps, operations or processes of different ones of the methods described above may also be executed by programmed computers, processors or other programmable hardware components. Examples may also cover program storage devices, such as digital data storage media, which are machine-, processor- or computer-readable and encode and/or contain machine-executable, processor-executable or computer-executable programs and instructions. Program storage devices may include or be digital storage devices, magnetic storage media such as magnetic disks and magnetic tapes, hard disk drives, or optically readable digital data storage media, for example. Other examples may also include computers, processors, control units, (field) programmable logic arrays ((F)PLAs), (field) programmable gate arrays ((F)PGAs), graphics processor units (GPU), application-specific integrated circuits (ASICs), integrated circuits (ICs) or system-on-a-chip (SoCs) systems programmed to execute the steps of the methods described above.

It is further understood that the disclosure of several steps, processes, operations or functions disclosed in the description or claims shall not be construed to imply that these operations are necessarily dependent on the order described, unless explicitly stated in the individual case or necessary for technical reasons. Therefore, the previous description does not limit the execution of several steps or functions to a certain order. Furthermore, in further examples, a single step, function, process or operation may include and/or be broken up into several sub-steps, - functions, -processes or -operations.

If some aspects have been described in relation to a device or system, these aspects should also be understood as a description of the corresponding method. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

## Claims

1. An apparatus (100) for measuring a heart rate of a target in a field of view of a radar sensor, the apparatus (100) comprising processing circuitry (110) configured to:
determine a frequency spectrum (200-1) corresponding to time-domain data (130) based on a receive signal generated by the radar sensor;
determine a breathing rate frequency based on a maximum amplitude (210-1) of the frequency spectrum (200-1);
determine a plurality of section-frequency-spectra, each corresponding to a respective section of the time-domain data (130), based on the breathing rate frequency;
determine an average of the section-frequency-spectra to obtain an averaged-frequency-spectrum (200-2);
subtract the averaged-frequency-spectrum (200-2) from the frequency spectrum (200-1) to obtain a difference spectrum (200-3); and
estimate a heart rate of the target based on a maximum amplitude (230-1) of the difference spectrum (200-3).

2. The apparatus (100) of claim 1, wherein the processing circuitry (110) is configured to:
apply a high-pass filter for a processing of the time-domain data (130),
calculate an average amplitude of the high-pass filtered time-domain data to obtain a noise-level average, and
determine the frequency spectrum (200-1) if the noise-level average is under a pre-specified noise-level threshold.

3. The apparatus (100) of claim 1 or claim 2, wherein the processing circuitry (110) is configured to:
calculate a mean value of amplitude values of the time-domain data (130),
subtract the mean value from the amplitude values of the time-domain data (130) to generate mean-removed time-domain data, and
determine the frequency spectrum for the mean-removed time-domain data.

4. The apparatus (100) of any one of the previous claims, wherein the processing circuitry (110) is configured to:
perform one or more range Fourier Transformations of the time-domain data (130) to obtain one or more range buffers, each divided into a plurality of range bins,
for each range buffer, identify a range bin (422) associated with a maximum amplitude to determine a range-time dataset (430), and
determine the frequency spectrum for the range-time dataset (430) corresponding to the identified range bin (422).

5. The apparatus (100) of claim 4, wherein the processing circuitry (110) is configured to:
check whether or not a peak-to-peak value for the range-time dataset (430)is within pre-specified limits based on an average of a plurality of previous peak-to-peak values in the range-time dataset (430), and
determine the frequency spectrum for the range-time dataset (430) if the peak-to-peak value is within its pre-specified limits.

6. The apparatus (100) of claim 4 or claim 5, wherein the processing circuitry (110) is configured to:
determine a plurality of derivative values corresponding to a rate of change of a range at a respective time of the range-time dataset (430),
determine a variance of the plurality of derivative values,
check that each of the derivative values is within a threshold determined by the variance, and
determine the frequency spectrum (200-1) for the range-time dataset (430) if each derivative value is within the threshold.

7. The apparatus (100) of any one of the previous claims, wherein the processing circuitry (110) is configured to determine the frequency spectrum (200-1) for the time-domain data (130) and to subsequently apply a finite-impulse-response filter to the amplitude values of the frequency spectrum (200-1).

8. The apparatus (100) of any one of the previous claims, wherein the processing circuitry (110) is configured to determine the breathing rate frequency by detection of a first frequency corresponding to a maximum amplitude (210-1) value of the frequency spectrum (200-1) and detection of a second frequency corresponding to a local maximum (210-2) of amplitude values in the frequency spectrum (200-1) within a pre-specified frequency range from twice the first frequency.

9. The apparatus (100) of claim 8, wherein the processing circuitry (110) is configured to verify that a maximum amplitude (220-1) value of the averaged-frequency-spectrum (200-2) corresponds to a frequency that is within a pre-specified frequency range from the breathing rate frequency.

10. The apparatus (100) of claim 9, wherein for the case that the maximum amplitude (220-1) value of the averaged-frequency-spectrum (200-2) corresponds to a frequency that is not within the pre-specified frequency range from the breathing rate frequency, the processing circuitry (110) is configured to:
determine a new breathing rate frequency based on a neighboring local maximum of amplitude values in the frequency spectrum (200-1),
obtain a new averaged-frequency-spectrum based on the new breathing rate frequency,
verify that the maximum amplitude value of the new averaged-frequency-spectrum corresponds to a frequency that is within the pre-specified frequency range from the new breathing rate frequency.

11. The apparatus (100) of any one of the previous claims, wherein the processing circuitry (110) is configured to:
perform a first correlation operation for the frequency spectrum (200-1) to determine a correlated frequency spectrum (730-3a) and a second correlation operation for the averaged-frequency-spectrum (200-2) to determine a correlated averaged-frequency-spectrum (730-3b),
wherein each correlation operation is between an original version (730-1) and a windowed version (730-2) of the respective spectrum,
wherein the respective windowed version (730-2) is altered by applying a first Gaussian window (732-1a; 732-1b) centered on a first frequency corresponding to a maximum amplitude (210-1; 220-1) of the spectrum, applying a second Gaussian window (732-2a; 732-2b) centered on a second frequency that is two times the first frequency, applying a third Gaussian window (732-3a; 732-3b) centered on a third frequency that is three times the first frequency, and nullifying all amplitude values outside of the three Gaussian windows; and
subtract the correlated averaged-frequency-spectrum (730-3b) from the correlated frequency spectrum (730-3a) to obtain the difference spectrum (200-3).

12. The apparatus (100) of any one of the previous claims, wherein the processing circuitry (110) is configured to perform multiple heart rate estimations, wherein each estimation is applied to an iteration of a recursive model for obtaining a refined heart rate estimate.

13. A radar system (900), comprising:
an apparatus (910) according to any one of claims 1 to 12; and
a radar sensor (920) configured to generate the receive signal.

14. A method (1000) for measuring a heart rate of a target in a field of view of a radar sensor, the method comprising:
determining a frequency spectrum (1010) corresponding to time-domain data based on a receive signal generated by the radar sensor;
determining a breathing rate frequency (1020) based on a maximum amplitude of the frequency spectrum;
determining a plurality of section-frequency-spectra (1030), each corresponding to a respective section of the time-domain data, based on the breathing rate frequency;
determining an average (1040) of the section-frequency-spectra to obtain an averaged-frequency-spectrum;
subtracting (1050) the averaged-frequency-spectrum from the frequency spectrum to obtain a difference spectrum; and
estimating (1060) a heart rate of the target based on a maximum amplitude of the difference spectrum,
wherein the method is implemented by a processing circuitry.

15. A program having a program code for performing the method according to claim 14 when the program is executed on a processor or programmable hardware.

## Patentansprüche

1. Vorrichtung (100) zum Messen einer Herzfrequenz eines Ziels in einem Sichtfeld eines Radarsensors, wobei die Vorrichtung (100) eine Verarbeitungsschaltungsanordnung (110) umfasst, die ausgelegt ist zum:
Bestimmen eines Frequenzspektrums (200-1), das Zeitdomänendaten (130) entspricht, basierend auf einem Empfangssignal, das durch den Radarsensor generiert wird;
Bestimmen einer Atemfrequenz basierend auf einer maximalen Amplitude (210-1) des Frequenzspektrums (200-1);
Bestimmen einer Mehrzahl von Teilfrequenzspektren, die jeweils einem jeweiligen Teil der Zeitdomänendaten (130) entsprechen, basierend auf der Atemfrequenz;
Bestimmen eines Mittelwerts der Teilfrequenzspektren, um ein gemitteltes Frequenzspektrum zu erhalten (200-2);
Subtrahieren des gemittelten Frequenzspektrums (200-2) von dem Frequenzspektrum (200-1), um ein Differenzspektrum (200-3) zu erhalten; und
Schätzen einer Herzfrequenz des Ziels basierend auf einer maximalen Amplitude (230-1) des Differenzspektrums (200-3).

2. Vorrichtung (100) nach Anspruch 1, wobei die Verarbeitungsschaltungsanordnung (110) ausgelegt ist zum:
Anwenden eines Hochpassfilters zur Verarbeitung der Zeitdomänendaten (130),
Berechnen einer mittleren Amplitude der hochpassgefilterten Zeitdomänendaten, um einen Rauschpegelmittelwert zu erhalten, und
Bestimmen des Frequenzspektrums (200-1), wenn der Rauschpegelmittelwert unter einem vorgegebenen Rauschpegelschwellenwert liegt.

3. Vorrichtung (100) nach Anspruch 1 oder Anspruch 2, wobei die Verarbeitungsschaltungsanordnung (110) ausgelegt ist zum:
Berechnen eines Mittelwerts von Amplitudenwerten der Zeitdomänendaten (130),
Subtrahieren des Mittelwerts von den Amplitudenwerten der Zeitdomänendaten (130), um Zeitdomänendaten mit entferntem Mittelwert zu generieren, und
Bestimmen des Frequenzspektrums für die Zeitdomänendaten mit entferntem Mittelwert.

4. Vorrichtung (100) nach einem der vorhergehenden Ansprüche, wobei die Verarbeitungsschaltungsanordnung (110) ausgelegt ist zum:
Durchführen einer oder mehrerer Bereichs-Fourier-Transformationen der Zeitdomänendaten (130), um einen oder mehrere Bereichspuffer zu erhalten, die jeweils in eine Mehrzahl von Bereichs-Bins unterteilt sind,
für jeden Bereichspuffer Identifizieren eines Bereichs-Bins (422), das mit einer maximalen Amplitude assoziiert ist, um einen Bereichs-Zeit-Datensatz (430) zu bestimmen, und
Bestimmen des Frequenzspektrums für den Bereichs-Zeit-Datensatz (430), der dem identifizierten Bereichs-Bin (422) entspricht.

5. Vorrichtung (100) nach Anspruch 4, wobei die Verarbeitungsschaltungsanordnung (110) ausgelegt ist zum:
Prüfen, ob ein Spitze-Spitze-Wert für den Bereichs-Zeit-Datensatz (430) innerhalb vorgegebener Grenzen liegt oder nicht, basierend auf einem Mittelwert einer Mehrzahl von vorherigen Spitze-Spitze-Werten in dem Bereichs-Zeit-Datensatz (430), und
Bestimmen des Frequenzspektrums für den Bereichs-Zeit-Datensatz (430), wenn der Spitze-Spitze-Wert innerhalb seiner vorgegebenen Grenzen liegt.

6. Vorrichtung (100) nach Anspruch 4 oder Anspruch 5, wobei die Verarbeitungsschaltungsanordnung (110) ausgelegt ist zum:
Bestimmen einer Mehrzahl von Ableitungswerten, die einer Änderungsrate eines Bereichs zu einer jeweiligen Zeit des Bereichs-Zeit-Datensatzes entsprechen (430),
Bestimmen einer Varianz der Mehrzahl von Ableitungswerten,
Prüfen, ob jeder der Ableitungswerte innerhalb eines Schwellenwerts liegt, der durch die Varianz bestimmt wird, und
Bestimmen des Frequenzspektrums (200-1) für den Bereichs-Zeit-Datensatz (430), wenn jeder Ableitungswert innerhalb des Schwellenwerts liegt.

7. Vorrichtung (100) nach einem der vorhergehenden Ansprüche, wobei die Verarbeitungsschaltungsanordnung (110) dazu ausgelegt ist, das Frequenzspektrum (200-1) für die Zeitdomänendaten (130) zu bestimmen und anschließend ein Filter mit endlicher Impulsantwort auf die Amplitudenwerte des Frequenzspektrums (200-1) anzuwenden.

8. Vorrichtung (100) nach einem der vorhergehenden Ansprüche, wobei die Verarbeitungsschaltungsanordnung (110) dazu ausgelegt ist, die Atemfrequenz durch Detektieren einer ersten Frequenz, die einem maximalen Amplitudenwert (210-1) des Frequenzspektrums (200-1) entspricht, und Detektieren einer zweiten Frequenz, die einem lokalen Maximum (210-2) von Amplitudenwerten in dem Frequenzspektrum (200-1) entspricht, innerhalb eines vorgegebenen Frequenzbereichs von dem Doppelten der ersten Frequenz zu bestimmen.

9. Vorrichtung (100) nach Anspruch 8, wobei die Verarbeitungsschaltungsanordnung (110) dazu ausgelegt ist, zu verifizieren, dass ein maximaler Amplitudenwert (220-1) des gemittelten Frequenzspektrums (200-2) einer Frequenz entspricht, die innerhalb eines vorgegebenen Frequenzbereichs von der Atemfrequenz liegt.

10. Vorrichtung (100) nach Anspruch 9, wobei für den Fall, dass der maximale Amplitudenwert (220-1) des gemittelten Frequenzspektrums (200-2) einer Frequenz entspricht, die nicht innerhalb des vorgegebenen Frequenzbereichs von der Atemfrequenz liegt, die Verarbeitungsschaltungsanordnung (110) ausgelegt ist zum:
Bestimmen einer neuen Atemfrequenz basierend auf einem benachbarten lokalen Maximum von Amplitudenwerten in dem Frequenzspektrum (200-1),
Erhalten eines neuen gemittelten Frequenzspektrums basierend auf der neuen Atemfrequenz,
Verifizieren, dass der maximale Amplitudenwert des neuen gemittelten Frequenzspektrums einer Frequenz entspricht, die innerhalb des vorgegebenen Frequenzbereichs von der neuen Atemfrequenz liegt.

11. Vorrichtung (100) nach einem der vorhergehenden Ansprüche, wobei die Verarbeitungsschaltungsanordnung (110) ausgelegt ist zum:
Durchführen einer ersten Korrelationsoperation für das Frequenzspektrum (200-1), um ein korreliertes Frequenzspektrum (730-3a) zu bestimmen, und einer zweiten Korrelationsoperation für das gemittelte Frequenzspektrum (200-2), um ein korreliertes gemitteltes Frequenzspektrum (730-3b) zu bestimmen,
wobei jede Korrelationsoperation zwischen einer Originalversion (730-1) und einer gefensterten Version (730-2) des jeweiligen Spektrums erfolgt,
wobei die jeweilige gefensterte Version (730-2) abgeändert wird durch Anwenden eines ersten Gauß-Fensters (732-1a; 732-1b), das auf einer ersten Frequenz zentriert ist, die einer maximalen Amplitude (210-1; 220-1) des Spektrums entspricht, Anwenden eines zweiten Gauß-Fensters (732-2a; 732-2b), das auf einer zweiten Frequenz zentriert ist, die das Doppelte der ersten Frequenz ist, Anwenden eines dritten Gauß-Fensters (732-3a; 732-3b), das auf einer dritten Frequenz zentriert ist, die das Dreifache der ersten Frequenz ist, und Nullifizieren aller Amplitudenwerte außerhalb der drei Gauß-Fenster; und
Subtrahieren des korrelierten gemittelten Frequenzspektrums (730-3b) von dem korrelierten Frequenzspektrum (730-3a), um das Differenzspektrum (200-3) zu erhalten.

12. Vorrichtung (100) nach einem der vorhergehenden Ansprüche, wobei die Verarbeitungsschaltungsanordnung (110) dazu ausgelegt ist, mehrere Herzfrequenzschätzungen durchzuführen, wobei jede Schätzung auf eine Iteration eines rekursiven Modells zum Erhalten einer verfeinerten Herzfrequenzschätzung angewendet wird.

13. Radarsystem (900), umfassend:
eine Vorrichtung (910) nach einem der Ansprüche 1 bis 12; und
einen Radarsensor (920), der dazu ausgelegt ist, das Empfangssignal zu generieren.

14. Verfahren (1000) zum Messen einer Herzfrequenz eines Ziels in einem Sichtfeld eines Radarsensors, wobei das Verfahren umfasst:
Bestimmen eines Frequenzspektrums (1010), das Zeitdomänendaten entspricht, basierend auf einem Empfangssignal, das durch den Radarsensor generiert wird;
Bestimmen einer Atemfrequenz (1020) basierend auf einer maximalen Amplitude des Frequenzspektrums;
Bestimmen einer Mehrzahl von Teilfrequenzspektren (1030), die jeweils einem jeweiligen Teil der Zeitdomänendaten entsprechen, basierend auf der Atemfrequenz;
Bestimmen eines Mittelwerts (1040) der Teilfrequenzspektren, um ein gemitteltes Frequenzspektrum zu erhalten;
Subtrahieren (1050) des gemittelten Frequenzspektrums von dem Frequenzspektrum, um ein Differenzspektrum zu erhalten; und
Schätzen (1060) einer Herzfrequenz des Ziels basierend auf einer maximalen Amplitude des Differenzspektrums, wobei das Verfahren durch eine Verarbeitungsschaltungsanordnung implementiert wird.

15. Programm mit einem Programmcode zum Durchführen des Verfahrens nach Anspruch 14, wenn das Programm auf einem Prozessor oder einer programmierbaren Hardware ausgeführt wird.

## Revendications

1. Appareil (100) pour mesurer un rythme cardiaque d'une cible dans un champ de vision d'un capteur radar, l'appareil (100) comprenant un ensemble de circuits de traitement (110) configuré pour :
déterminer un spectre de fréquence (200-1) correspondant à des données de domaine temporel (130) basé sur un signal de réception généré par le capteur radar ;
déterminer une fréquence de rythme respiratoire basée sur une amplitude maximale (210-1) du spectre de fréquence (200-1) ;
déterminer une pluralité de spectres de fréquence de section, chacun correspondant à une section respective des données de domaine temporel (130), basé sur la fréquence de rythme respiratoire ;
déterminer une moyenne des spectres de fréquence de section pour obtenir un spectre de fréquence moyenné (200-2) ;
soustraire le spectre de fréquence moyenné (200-2) du spectre de fréquence (200-1) pour obtenir un spectre de différence (200-3) ; et
estimer un rythme cardiaque de la cible basé sur une amplitude maximale (230-1) du spectre de différence (200-3).

2. Appareil (100) selon la revendication 1, l'ensemble de circuits de traitement (110) étant configuré pour :
appliquer un filtre passe-haut pour un traitement des données de domaine temporel (130),
calculer une amplitude moyenne des données de domaine temporel filtrées passe-haut pour obtenir une moyenne de niveau de bruit, et
déterminer le spectre de fréquence (200-1) si la moyenne de niveau de bruit est sous un seuil de niveau de bruit pré-spécifié.

3. Appareil (100) selon la revendication 1 ou la revendication 2, l'ensemble de circuits de traitement (110) étant configuré pour :
calculer une valeur moyenne de valeurs d'amplitude des données de domaine temporel (130),
soustraire la valeur moyenne des valeurs d'amplitude des données de domaine temporel (130) pour générer des données de domaine temporel à moyenne supprimée, et
déterminer le spectre de fréquence pour les données de domaine temporel à moyenne supprimée.

4. Appareil (100) selon l'une quelconque des revendications précédentes, l'ensemble de circuits de traitement (110) étant configuré pour :
effectuer une ou plusieurs transformations de Fourier de distance des données de domaine temporel (130) pour obtenir un ou plusieurs tampons de distance, chacun divisé en une pluralité d'intervalles de distance,
pour chaque tampon de distance, identifier un intervalle de distance (422) associée à une amplitude maximale pour déterminer un jeu de données de temps de distance (430), et
déterminer le spectre de fréquence pour le jeu de données de temps de distance (430) correspondant à l'intervalle de distance identifiée (422).

5. Appareil (100) selon la revendication 4, l'ensemble de circuits de traitement (110) étant configuré pour :
vérifier si oui ou non une valeur crête-à-crête pour le jeu de données de temps de distance (430) est dans des limites pré-spécifiées basées sur une moyenne d'une pluralité de valeurs crête-à-crête précédentes dans le jeu de données de temps de distance (430), et
déterminer le spectre de fréquence pour le jeu de données de temps de distance (430) si la valeur crête-à-crête est dans ses limites pré-spécifiées.

6. Appareil (100) selon la revendication 4 ou la revendication 5, l'ensemble de circuits de traitement (110) étant configuré pour :
déterminer une pluralité de valeurs dérivées correspondant à un taux de changement d'une distance à un temps respectif du jeu de données de temps de distance (430),
déterminer une variance de la pluralité de valeurs dérivées,
vérifier que chacune des valeurs dérivées est en deçà d'un seuil déterminé par la variance, et
déterminer le spectre de fréquence (200-1) pour le jeu de données de temps de distance (430) si chaque valeur dérivée est en deçà du seuil.

7. Appareil (100) selon l'une quelconque des revendications précédentes, l'ensemble de circuits de traitement (110) étant configuré pour déterminer le spectre de fréquence (200-1) pour les données de domaine temporel (130) et pour appliquer subséquemment un filtre à réponse impulsionnelle finie aux valeurs d'amplitude du spectre de fréquence (200-1).

8. Appareil (100) selon l'une quelconque des revendications précédentes, l'ensemble de circuits de traitement (110) étant configuré pour déterminer la fréquence de rythme respiratoire par détection d'une première fréquence correspondant à une valeur d'amplitude maximale (210-1) du spectre de fréquence (200-1) et détection d'une deuxième fréquence correspondant à un maximum local (210-2) de valeurs d'amplitude dans le spectre de fréquence (200-1) dans une plage de fréquence pré-spécifiée de deux fois la première fréquence.

9. Appareil (100) selon la revendication 8, l'ensemble de circuits de traitement (110) étant configuré pour vérifier qu'une valeur d'amplitude maximale (220-1) du spectre de fréquence moyenné (200-2) correspond à une fréquence qui est dans une plage de fréquence pré-spécifiée de la fréquence de rythme respiratoire.

10. Appareil (100) selon la revendication 9, pour le cas où la valeur d'amplitude maximale (220-1) du spectre de fréquence moyenné (200-2) correspond à une fréquence qui n'est pas dans la plage de fréquence pré-spécifiée de la fréquence de rythme respiratoire, l'ensemble de circuits de traitement (110) étant configuré pour :
déterminer une nouvelle fréquence de rythme respiratoire basée sur un maximum local voisin de valeurs d'amplitude dans le spectre de fréquence (200-1),
obtenir un nouveau spectre de fréquence moyenné basé sur la nouvelle fréquence de rythme respiratoire,
vérifier que la valeur d'amplitude maximale du nouveau spectre de fréquence moyenné correspond à une fréquence qui est dans la plage de fréquence pré-spécifiée de la nouvelle fréquence de rythme respiratoire.

11. Appareil (100) selon l'une quelconque des revendications précédentes, l'ensemble de circuits de traitement (110) étant configuré pour :
effectuer une première opération de corrélation pour le spectre de fréquence (200-1) pour déterminer un spectre de fréquence corrélé (730-3a) et une seconde opération de corrélation pour le spectre de fréquence moyenné (200-2) pour déterminer un spectre de fréquence moyenné corrélé (730-3b),
chaque opération de corrélation étant entre une version originale (730-1) et une version fenêtrée (730-2) du spectre respectif,
la version fenêtrée respective (730-2) étant altérée par application d'une première fenêtre gaussienne (732-1a, 732-1b) centrée sur une première fréquence correspondant à une amplitude maximale (210-1 ; 220-1) du spectre, application d'une seconde fenêtre gaussienne (732-2a, 732-2b) centrée sur une deuxième fréquence qui est deux fois la première fréquence, application d'une troisième fenêtre gaussienne (732-3a, 732-3b) centrée sur une troisième fréquence qui est trois fois la première fréquence, et annulation de toutes les valeurs d'amplitude à l'extérieur des trois fenêtres gaussiennes, et
soustraire le spectre de fréquence moyenné corrélé (730-3b) du spectre de fréquence corrélé (730-3a) pour obtenir le spectre de différence (200-3).

12. Appareil (100) selon l'une quelconque des revendications précédentes, l'ensemble de circuits de traitement (110) étant configuré pour effectuer des estimations multiples de rythme cardiaque, chaque estimation étant appliquée à une itération d'un modèle récursif pour obtenir une estimation de rythme cardiaque affinée.

13. Système radar (900) comprenant :
un appareil (910) selon l'une quelconque des revendications 1 à 12 ; et
un capteur radar (920) configuré pour générer le signal de réception.

14. Procédé (1000) pour mesurer un rythme cardiaque d'une cible dans un champ de vision d'un capteur radar, le procédé comprenant :
la détermination d'un spectre de fréquence (1010) correspondant à des données de domaine temporel basé sur un signal de réception généré par le capteur radar ;
la détermination d'une fréquence de rythme respiratoire (1020) basée sur une amplitude maximale du spectre de fréquence ;
la détermination d'une pluralité de spectres de fréquence de section (1030), chacun correspondant à une section respective des données de domaine temporel, basé sur la fréquence de rythme respiratoire ;
la détermination d'une moyenne (1040) des spectres de fréquence de section pour obtenir un spectre de fréquence moyenné ;
la soustraction (1050) du spectre de fréquence moyenné du spectre de fréquence pour obtenir un spectre de différence ; et
l'estimation (1060) d'un rythme cardiaque de la cible basé sur une amplitude maximale du spectre de différence, le procédé étant mis en œuvre par un ensemble de circuits de traitement.

15. Programme ayant un code de programme pour effectuer le procédé selon la revendication 14 lorsque le programme est exécuté sur un processeur ou matériel programmable.
